# EUROPEAN PATENT SPECIFICATION

(11) **EP 3 074 088 B1**
(45) Date of publication and mention of the grant of the patent: **08.01.2020**
(21) Application number: 14812044.7
(22) Date of filing: 14.11.2014
(51) Int. Cl.: A61N 5/10, G01T 1/29, G01T 1/16

(54) **RADIATION BEAM MEASUREMENT NORMALIZATION**
STRAHLUNGSBÜNDELMESSUNGSNORMALISIERUNG
NORMALISATION DE LA MESURE DE FAISCEAU DE RADIATION

(30) Priority: 15.11.2013 US 201314081416
(43) Date of publication of application: 05.10.2016
(73) Proprietor: Sun Nuclear Corporation, Melbourne, FL 32940 (US)
(72) Inventor: HILDRETH, Jeffrey, L., Melbourne, FL 32940 (US); SIMON, Thomas, Rockledge, FL 32955 (US)
(74) Representative: Schulte, Drew Joseph
(86) International application number: PCT/US2014/065808
(87) International publication number: WO 2015/073899

(56) References cited:
- US-A- 5 627 367
- US-A1- 2011 022 360
- J U Wuerfel: "DOSE MEASUREMENTS IN SMALL FIELDS", MEDICAL PHYSICS INTERNATIONAL Journal, 1 January 2013 (2013-01-01), XP055500744, Retrieved from the Internet: URL:https://pdfs.semanticscholar.org/d661/ 3ad5807e4345d3ad689494b99d23939908ab.pdf [retrieved on 2018-08-20]

## Description

### TECHNICAL FIELD

The subject matter described herein relates to radiotherapy systems, and more particularly, to systems and methods for normalizing radiation beam measurements to compensate for variations in radiation output.

### BACKGROUND

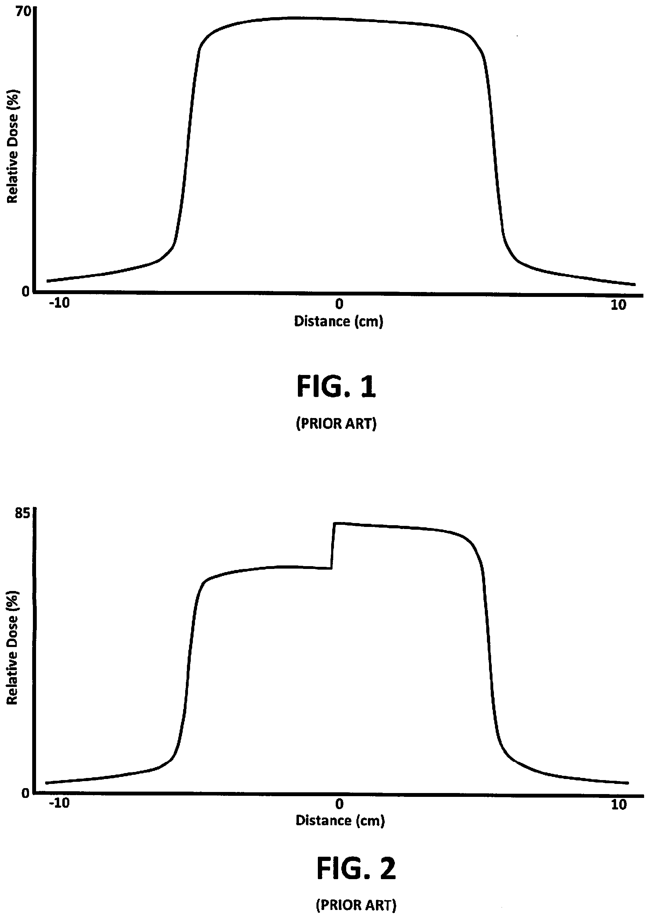

Linear accelerator (LINAC) relative dosimetry (beam scanning) involves acquiring a measurement of the radiation beam with a field detector and typically a reference detector. The measurement is acquired as two parameters are varied: the field detector's position and time. Using the measured signal from the field detector, a plot of the LINAC's radiation beam intensity versus position within that beam is possible, with an example shown in Figure 1.

Beam scanning often requires normalizing the measured signal from a field detector by the measured signal from a reference detector. This occurs because the output of the LINAC may change with time, sometimes abruptly. For example, an increase in the LINAC radiation dose rate during a scan results in an increase in the field detector's measured signal. If only a field detector is used, the beam scan will show a change in the beam intensity at that point in space that corresponds to when the LINAC's radiation output changed. This irregularity in the measurement is not indicative of the actual relative dose intensity of the radiation beam. In Figure 2, a repeat of the scan from Figure 1 is shown, but in which the LINAC's dose rate changed (increased) during the scan. This change caused the marked change in the amplitude of the beam's relative dose intensity.

Small changes in LINAC dose rate do not affect the radiation therapy treatment. The change in the dose rate effectively changes the amount of time in which the radiation dose is delivered, which is not important to the treatment parameters being measured. The more important parameter during beam scanning is the relative dose intensity of the beam. Using only a field detector may misrepresent this characteristic of the LINAC's radiation beam, as in Figure 2.

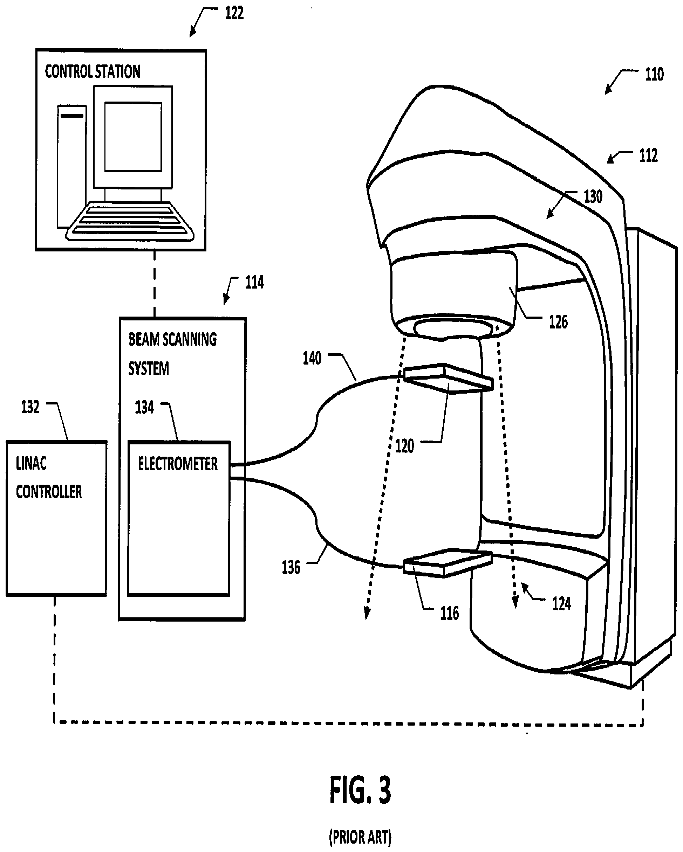

To correct for changes in a LINAC's dose rate, a reference detector is used to normalize dose rate changes. For example, referring to Figure 3, a radiotherapy system 110 includes a reference detector 120 that is stationary and located in the radiation beam 124. Considering the above example, when the LINAC 112 dose rate increased, it increases the measured signal of the field detector 116, which would otherwise misrepresent the relative dose intensity of the radiation beam 124 (as in Figure 2). However, the increase in the field detector's measured signal should be matched by a proportional increase in the reference detector's measured signal. A ratio of the field detector's measured signal to the reference detector's measured signal corrects for the changed dose rate that occurred during a scan. The measurement would then match that of Figure 1, correctly representing the beam's relative dose intensity. While this approach to beam measurement normalization is useful, further improvements are possible.

US 2011/022360 A1 discloses a three dimensional radiation measurement scanning system, which includes a circular drive operable with horizontal and vertical drives for moving a radiation detector through first, second and third orthogonal axes in a three dimensional scanning of the detector in a water tank. A reference detector is fixed for comparing its radiation field measurements with those of the scanned radiation detector. An offset mount carries the radiation detector allowing it to be extended beyond the circular ring gear during horizontal movement of the radiation detector and thus position the radiation detector at wall surfaces of the water tank.

J U Wuerfel: "DOSE MEASUREMENTS IN SMALL FIELDS", MEDICAL PHYSICS INTERNATIONAL Journal, 1 January 2013 (2013-01-01), XP055500744, relates to a radiation therapy device using small irradiation fields.

The invention is defined in claims 1 and 7. Further aspects and preferred embodiments are defined in the dependent claims. Aspects, embodiments and examples of the present disclosure which do not fall under the scope of the appended claims do not form part of the invention and are merely provided for illustrative purposes.

US 2011/022360 A1 discloses a three dimensional radiation measurement scanning system, which includes a circular drive operable with horizontal and vertical drives for moving a radiation detector through first, second and third orthogonal axes in a three dimensional scanning of the detector in a water tank. A reference detector is fixed for comparing its radiation field measurements with those of the scanned radiation detector. An offset mount carries the radiation detector allowing it to be extended beyond the circular ring gear during horizontal movement of the radiation detector and thus position the radiation detector at wall surfaces of the water tank.

J U Wuerfel: "DOSE MEASUREMENTS IN SMALL FIELDS", MEDICAL PHYSICS INTERNATIONAL Journal, 1 January 2013 (2013-01-01), XP055500744, relates to a radiation therapy device using small irradiation fields.

### SUMMARY

In one aspect, a radiotherapy system includes computing hardware configured to perform operations that include receiving a beam measurement signal from a field detector and a reference signal from a reference detector. The field detector is positioned within the radiation beam and generates a beam measurement signal representative of a radiation beam directed from a head of a radiotherapy device during operation of the radiotherapy device. The reference detector is positioned outside of the radiation beam during operation of the radiotherapy device and generates a reference signal. The operations also include normalizing the beam measurement signal based on the reference signal, and outputting a normalized beam measurement. The radiotherapy system also includes the radiotherapy device. The radiotherapy device includes a movable gantry that carries the head, and the reference detector is carried on the movable gantry so as to move therewith. The head directs the radiation beam away from a first side of the movable gantry and the reference detector is carried on a second side of the movable gantry opposite the first side

In another interrelated aspect, a method for providing a normalized radiation beam measurement for a radiotherapy device includes measuring a beam measurement signal representative of ionizing radiation within a radiation beam generated by the radiotherapy device, measuring a reference signal representative of ionizing radiation outside of the radiation beam, normalizing the beam measurement signal based on the reference signal to generate the normalized radiation beam measurement, and outputting the normalized radiation beam measurement.

In optional variations, one or more of the following features can be included in any feasible combination. The radiotherapy device can include a linear accelerator (LINAC). The reference detector can be positioned to measure head scatter from the LINAC. The normalized beam measurement can include a measurement of a relative intensity of the radiation. beam versus position. A radiotherapy system can further include the field detector, the reference detector, and at least one electrometer configured to measure the beam measurement and reference signals from the field detector and the reference detector and to output respective measured beam measurement and reference signals to the computing hardware. Such an electrometer can be connected to the field detector and the reference detector by respective cables. The field detector and the reference detector can be configured to detect ionizing radiation, and the beam measurement and reference signals can be representative of an acquired charge resulting from detecting the ionizing radiation at the field detector and the reference detector, respectively.

Systems and methods consistent with this approach are described as well as articles that comprise a tangibly embodied machine-readable medium operable to cause one or more machines (e.g., computers, etc.) to result in operations described herein. Similarly, computer systems are also described that may include a processor and a memory coupled to the processor. The memory may include one or more programs that cause the processor to perform one or more of the operations described herein.

The details of one or more variations of the subject matter described herein are set forth in the accompanying drawings and the description below. Other features and advantages of the subject matter described herein will be apparent from the description and drawings, and from the claims. The invention is defined in claim 1. Further aspects and preferred embodiments are defined in the dependent claims. Aspects, embodiments and examples of the present disclosure which do not fall under the scope of the appended claims do not form part of the invention and are merely provided for illustrative purposes.

### DESCRIPTION OF DRAWINGS

The accompanying drawings, which are incorporated in and constitute a part of this specification, show certain aspects of the subject matter disclosed herein and, together with the description, help explain some of the principles associated with the disclosed implementations. In the drawings,
Figure 1 is graph of a normalized beam measurement;
Figure 2 is a graph of a non-normalized beam measurement;
Figure 3 is schematic view of a radiotherapy system configured for beam measurement normalization; and
Figure 4 is a schematic view of a radiotherapy system configured for beam measurement normalization, according to an embodiment of the present invention.

When practical, similar reference numbers denote similar structures, features, or elements.

### DETAILED DESCRIPTION

Implementations of the current subject matter can provide improved approaches for radiation beam measurement normalization. A radiotherapy system includes a radiotherapy device, such as a LINAC, operable to direct a radiation beam from a head thereof during operation, a field detector positioned to be within the radiation beam during operation of the radiotherapy device and operable to generate a beam measurement signal, a reference detector positioned to be outside of the radiation beam during operation of the radiotherapy device and operable to generate a reference signal, and at least one computer in signal communication with the field detector and the reference detector and configured with software to normalize the beam measurement signal based on the reference signal and to output a normalized beam measurement.

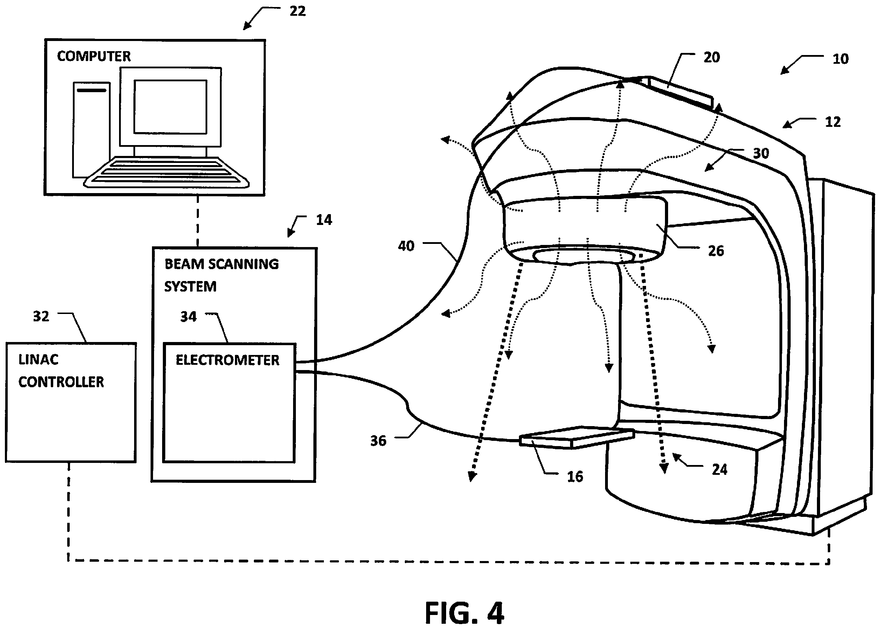

Referring to Figure 4, according to an embodiment of the present invention, a representative radiotherapy system 10 includes a radiotherapy device 12, a beam scanning system 14 receiving signals from field and reference detectors 16, 20, and a computer 22 in signal communication with the detectors 16, 20 via the beam scanning system 14. The radiotherapy device 12 is operable to direct a radiation beam 24 from a head 26 thereof, the operation also producing head scatter 30. The field detector 16 is arranged so as to be in the radiation beam 24 and operable to generate a beam measurement signal, while the reference detector 20 is positioned to be outside of the radiation beam and operable to generate a reference signal based on the head scatter 30. The computer 22 is configured with software to normalize the beam measurement signal based on the reference signal and to output a normalized beam measurement.

The radiotherapy device 12 is advantageously a linear accelerator (LINAC) used in connection with beam scanning during operation (i.e., measuring the relative dose intensity). Generation and movement of the radiation beam and rotation of the head 26 are performed by a LINAC controller 32 capable of sending and receiving control signals to the LINAC 12 to effect device operation.

The beam scanning system 14 includes an electrometer 34 connected to the field and reference detectors 16, 20 by respective cables 36, 40. The acquired electrical charge of the detectors 16, 20 is thereby supplied to the electrometer 34 for measurement, with the measured values being output to the computer 22. If it is necessary to provide a polarizing voltage to the field and reference detectors 16, 20, the polarizing voltage is provided via their respective cables 36, 40 and the electrometer 34.

The field and reference detectors 16, 20 are both configured to detect ionizing radiation which, as indicated above, will alter the acquired charge of the detectors. The field detector 16 is placed where it will be in the radiation beam 24 emitted by the LINAC 12 during operation. The reference detector 20 is placed at location that will be outside of the radiation beam 24 during operation, but still exposed to head scatter 30; for instance, on top of the head 26. In a LINAC, the head scatter is produced by interactions between the LINAC-generated beam and components of the LINAC head. Notably, the depicted beam 24 and head scatter 30 are provided for illustrative purposes, and not intended to reflect actual geometries).

As used herein, the term "computer" (e.g., the computer 22) is used to broadly indicate at least one hardware processor capable of executing program instructions, machine-readable memory media for storing such instructions and related data used and/or generated during the operation thereof. Typically, a computer will also include one or more input/output devices, such as video displays, keyboards, pointing devices and the like. A "computer" can encompass multiple, physically discrete and separately housed processors, memory media and the like that are in signal communication (for instance, via a network) to perform the enumerated functions. Additionally, the computer 22 can be structurally and/or functionally integrated with some or all of the beam scanning system 14 and/or LINAC controller 32.

The computer 22 is configured with software to output a normalized beam measurement based on the beam measurement signal and the reference signal. In particular, the software directs the calculation of the ratio of the beam measurement signal from the field detector and the reference signal from the reference detector. This effectively normalizes the beam measurement since changes in dose rate will affect the numerator and the denominator in the same proportion. This is because the dose rate of the beam and the head scatter rate are linearly proportional. Minor changes in beam shape, on the other hand, will not affect the head scatter rate.

The present invention can offer several advantages over normalization that requires locating the reference detector within the radiation beam. For example, accurate beam normalization is still possible even where the radiation beam size too small to accommodate both a field detector and a reference detector. Also, it is not necessary to reposition the reference detector as the beam size changes. The reference detector can be securely mounted in a fixed location, making unintentional movement of the reference detector less likely. Additionally, as mentioned above, the reference detector will not be sensitive to fluctuations in beam shape during scanning, which might otherwise affect the reference detector independently of the field detector.

One or more aspects or features of the subject matter described herein can be realized in digital electronic circuitry, integrated circuitry, specially designed application specific integrated circuits (ASICs), field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device.

These computer programs, which can also be referred to as programs, software, software applications, applications, components, or code, include machine instructions for a programmable processor, and can be implemented in a high-level procedural language, an object-oriented programming language, a functional programming language, a logical programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including, but not limited to, acoustic, speech, or tactile input. Other possible input devices include, but are not limited to, touch screens or other touch-sensitive devices such as single or multi-point resistive or capacitive trackpads, voice recognition hardware and software, optical scanners, optical pointers, digital image capture devices and associated interpretation software, and the like. A computer remote from an analyzer can be linked to the analyzer over a wired or wireless network to enable data exchange between the analyzer and the remote computer (e.g. receiving data at the remote computer from the analyzer and transmitting information such as calibration data, operating parameters, software upgrades or updates, and the like) as well as remote control, diagnostics, etc. of the analyzer.

In the descriptions above and in the claims, phrases such as "at least one of' or "one or more of' may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

The subject matter described herein can be embodied in systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and subcombinations of the disclosed features and/or combinations and subcombinations of several further features disclosed above. In addition, the logic flows depicted in the accompanying figures and/or described herein do not necessarily require the particular order shown, or sequential order, to achieve desirable results. Other implementations may be within the scope of the following claims.

## Claims

1. A radiotherapy system (10) comprising:
a radiotherapy device (12) comprising a head (26) and a moveable gantry that carries the head (26);
a reference detector (20);
computing hardware configured to perform operations comprising:
receiving a beam measurement signal from a field detector (16) and a reference signal from the reference detector (20), the field detector (16) generating a beam measurement signal representative of a radiation beam (24) directed from the head (26) of the radiotherapy device (12) during operation of the radiotherapy device (12) and being positioned within the radiation beam (24), the reference detector (20) generating a reference signal;
normalizing the beam measurement signal based on the reference signal, and
outputting a normalized beam measurement; wherein
the reference detector (20) is positioned outside of the radiation beam (24) during operation of the radiotherapy device (12), **characterized in that** the reference detector (20) is carried on the moveable gantry so as to move therewith, wherein the head (26) is configured to direct the radiation beam (24) away from a first side of the movable gantry and the reference detector (20) is carried on a second side of the movable gantry opposite the first side.

2. The radiotherapy system (10) of claim 1, wherein the reference detector (20) is positioned to measure head scatter (30).

3. The radiotherapy system (10) of claims 1 and 2, wherein the normalized beam measurement comprises a measurement of a relative intensity of the radiation beam (24) versus position.

4. The radiotherapy system (10) of anyone of claims 1 to 3, further comprising: the field detector (16).

5. The radiotherapy system (10) of anyone of claims 1 to 4, wherein the field detector (16) and the reference detector (20) are configured to detect ionizing radiation and wherein the beam measurement and reference signals are representative of an acquired charge resulting from detecting the ionizing radiation at the field detector (16) and the reference detector (20), respectively.

6. The radiotherapy system (10) of anyone of claims 1 to 5, wherein the computing hardware comprises a programmable processor and a machine-readable medium storing instructions that, when executed by the programmable processor, cause the programmable processor to perform at least some of the operations.

7. A method for providing a normalized radiation beam measurement for a radiotherapy device (12), the method comprising:
measuring a beam measurement signal representative of ionizing radiation within a radiation beam (24) directed from a head (26) of the radiotherapy device (12);
measuring a reference signal representative of ionizing radiation;
normalizing the beam measurement signal based on the reference signal to generate the normalized radiation beam measurement; and
outputting the normalized radiation beam measurement;
**characterized in that**
the reference signal is representative of ionizing radiation outside of the radiation beam (24), wherein the reference signal is generated by a reference detector (20) positioned outside of the radiation beam (24) during operation of the radiotherapy device (12), wherein the radiotherapy device (12) includes a moveable gantry that carries the head (26), and the reference detector (20) is carried on the moveable gantry so as to move therewith, wherein the head (26) directs the radiation beam (24) away from a first side of the movable gantry and the reference detector (20) is carried on a second side of the movable gantry opposite the first side.

8. The method of claim 7, wherein the beam measurement signal is generated by a field detector (16) positioned within the radiation beam (24) during operation of the radiotherapy device (12).

9. The method of claim 8, wherein the reference detector (20) is positioned to receive head scatter (30) from the radiotherapy device (12).

10. The method of anyone of claims 7 to 9, wherein the radiotherapy device (12) comprises a linear accelerator (LINAC).

11. The method of anyone of claims 7 to 10, wherein the normalized beam measurement comprises a measurement of a relative intensity of the radiation beam (24) versus position and/or
the measuring of the beam measurement and reference signals from the field detector (16) and the reference detector (20) are performed using an electrometer that outputs the beam measurement and reference signals.

12. A computer program product comprising a machine-readable medium storing instructions to cause the system of claim 1 to perform operations comprising:
receiving a beam measurement signal from a field detector (16) and a reference signal from a reference detector (20), the field detector (16) generating a beam measurement signal representative of a radiation beam (24) directed from a head of a radiotherapy device during operation of the radiotherapy device (12) and being positioned within the radiation beam (24),
the reference detector (20) generating a reference signal;
normalizing the beam measurement signal based on the reference signal, and
outputting a normalized beam measurement;
**characterized in that**
the reference detector (20) is positioned outside of the radiation beam (24) during operation of the radiotherapy device (12), wherein the radiotherapy device (12) includes a moveable gantry that carries the head (26), and the reference detector (20) is carried on the moveable gantry so as to move therewith, wherein the head (26) directs the radiation beam (24) away from a first side of the movable gantry and the reference detector (20) is carried on a second side of the movable gantry opposite the first side.

## Patentansprüche

1. Strahlentherapiesystem (10), umfassend:
eine Strahlentherapievorrichtung (12), die einen Kopf (26) und
ein bewegliches Gerüst, das den Kopf (26) trägt, umfasst;
eine Referenzdetektor (20);
Rechenhardware, die dazu konfiguriert ist, Vorgänge durchzuführen, die Folgendes umfassen:
Empfangen eines Bündelmessungssignals von einem Felddetektor (16) und eines Referenzsignals von dem Referenzdetektor (20), wobei der Felddetektor (16) ein Bündelmessungssignal generiert, das repräsentativ für ein Strahlungsbündel (24) ist, das während des Betriebs der Strahlentherapievorrichtung (12) von dem Kopf (26) der Strahlentherapievorrichtung (12) gerichtet ist, und innerhalb des Strahlungsbündels (24) positioniert ist, wobei der Referenzdetektor (20) ein Referenzsignal generiert;
Normalisieren des Bündelmessungssignals auf Grundlage des Referenzsignals; und
Ausgeben einer normalisierten Bündelmessung;
wobei der Referenzdetektor (20) während des Betriebs der Strahlentherapievorrichtung (12) außerhalb des Strahlungsbündels (24) positioniert ist,
**dadurch gekennzeichnet, dass** der Referenzdetektor (20) auf dem beweglichen Gestell so getragen wird, dass er sich mit diesem bewegt, wobei der Kopf (26) dazu konfiguriert ist, das Strahlungsbündel (24) von einer ersten Seite des beweglichen Gestells weg zu richten und der Referenzdetektor (20) auf einer zweiten Seite des beweglichen Gestells gegenüber der ersten Seite getragen wird.

2. Strahlentherapiesystem (10) nach Anspruch 1, wobei der Referenzdetektor (20) positioniert ist, um Streustrahlung (30) des Kopfs zu messen.

3. Strahlentherapiesystem (10) nach Ansprüchen 1 und 2, wobei die normalisierte Bündelmessung eine Messung einer relativen Intensität des Strahlungsbündels (24) im Vergleich zu der Position umfasst.

4. Strahlentherapiesystem (10) nach einem der Ansprüche 1 bis 3, ferner umfassend: den Felddetektor (16).

5. Strahlentherapiesystem (10) nach einem der Ansprüche 1 bis 4, wobei der Felddetektor (16) und der Referenzdetektor (20) dazu konfiguriert sind, Ionisierungsstrahlung zu detektieren, und wobei die Bündelmessungs- und Referenzsignale repräsentativ für eine erworbene Ladung sind, die aus dem Detektieren der Ionisierungsstrahlung an dem Felddetektor (16) bzw. dem Referenzdetektor (20) resultiert.

6. Strahlentherapiesystem (10) nach einem der Ansprüche 1 bis 5, wobei die Rechenhardware einen programmierbaren Prozessor und ein maschinenlesbares Medium umfasst, das Anweisungen speichert, die bei Ausführung durch den programmierbaren Prozessor den programmierbaren Prozessor dazu veranlassen, mindestens einige der Vorgänge durchzuführen.

7. Verfahren zum Bereitstellen einer normalisierten Strahlungsbündelmessung für eine Strahlentherapievorrichtung (12), wobei das Verfahren Folgendes umfasst:
Messen eines Bündelmessungssignals, das repräsentativ für Ionisierungsstrahlung innerhalb eines Strahlungsbündels (24) ist, das von einem Kopf (26) der Strahlentherapievorrichtung (12) gerichtet ist;
Messen eines Referenzsignals, das repräsentativ für die Ionisierungsstrahlung ist;
Normalisieren des Bündelmessungssignals auf Grundlage des Referenzsignals, um die normalisierte Strahlungsbündelmessung zu generieren; und
Ausgeben der normalisierten Strahlungsbündelmessung;
**dadurch gekennzeichnet, dass**
das Referenzsignal repräsentativ für die Ionisierungsstrahlung außerhalb des Strahlungsbündels (24) ist, wobei das Referenzsignal durch einen Referenzdetektor (20) generiert wird, der während des Betrieb der Strahlentherapievorrichtung (12) außerhalb des Strahlungsbündels (24) positioniert ist, wobei die Strahlentherapievorrichtung (12) ein bewegliches Gestell beinhaltet, das den Kopf (26) trägt, und der Referenzdetektor (20) so auf dem beweglichen Gestell getragen wird, dass er sich mit diesem bewegt, wobei der Kopf (26) das Strahlungsbündel (24) von einer ersten Seite des beweglichen Gestells weg richtet und der Referenzdetektor (20) auf einer zweiten Seite des beweglichen Gestells gegenüber der ersten Seite getragen wird.

8. Verfahren nach Anspruch 7, wobei das Bündelmessungssignal durch einen Felddetektor (16) generiert wird, der während des Betriebs der Strahlentherapievorrichtung (12) innerhalb des Strahlungsbündels (24) positioniert ist.

9. Verfahren nach Anspruch 8, wobei der Referenzdetektor (20) positioniert ist, um Streustrahlung (30) des Kopfs von der Strahlentherapievorrichtung (12) zu empfangen.

10. Verfahren nach einem der Ansprüche 7 bis 9, wobei die Strahlentherapievorrichtung (12) einen Linearbeschleuniger (LINAC) umfasst.

11. Verfahren nach einem der Ansprüche 7 bis 10, wobei die normalisierte Bündelmessung eine Messung einer relativen Intensität des Strahlungsbündels (24) im Vergleich zu der Position umfasst und/oder
das Messen der Bündelmessungs- und Referenzsignale von dem Felddetektor (16) und dem Referenzdetektor (20) unter Verwendung eines Elektrometers durchgeführt wird, das die Bündelmessungs- und Referenzsignale ausgibt.

12. Computerprogrammprodukt, umfassend ein maschinenlesbares Medium, das Anweisungen speichert, die das System des Anspruchs 1 dazu veranlassen, Vorgänge durchzuführen, die Folgendes umfassen:
Empfangen eines Bündelmessungssignals von einem Felddetektor (16) und eines Referenzsignals von dem Referenzdetektor (20), wobei der Felddetektor (16) ein Bündelmessungssignal generiert, das repräsentativ für ein Strahlungsbündel (24) ist, das während des Betriebs der Strahlentherapievorrichtung (12) von einem Kopf einer Strahlentherapievorrichtung gerichtet ist, und innerhalb des Strahlungsbündels (24) positioniert ist,
wobei der Referenzdetektor (20) ein Referenzsignal generiert;
Normalisieren des Bündelmessungssignals auf Grundlage des Referenzsignals, und
Ausgeben einer normalisierten Bündelmessung;
**dadurch gekennzeichnet, dass**
der Referenzdetektor (20) während des Betriebs der Strahlentherapievorrichtung (12) außerhalb des Strahlungsbündels (24) positioniert ist, wobei die Strahlentherapievorrichtung (12) ein bewegliches Gestell beinhaltet, das den Kopf (26) trägt, und der Referenzdetektor (20) auf dem beweglichen Gestell so getragen wird, dass er sich mit diesem bewegt, wobei der Kopf (26) das Strahlungsbündel (24) von einer ersten Seite des beweglichen Gestells weg richtet und der Referenzdetektor (20) auf einer zweiten Seite des beweglichen Gestells gegenüber der ersten Seite getragen wird.

## Revendications

1. Système de radiothérapie (10) comprenant :
un dispositif de radiothérapie (12) comprenant une tête (26) et un portique mobile qui porte la tête (26) ;
un détecteur de référence (20) ;
un matériel informatique configuré pour effectuer des opérations comprenant :
la réception d'un signal de mesure de faisceau provenant d'un détecteur de champ (16) et d'un signal de référence provenant du détecteur de référence (20), le détecteur de champ (16) générant un signal de mesure de faisceau représentatif d'un faisceau de radiation (24) dirigé depuis la tête (26) du dispositif de radiothérapie (12) pendant le fonctionnement du dispositif de radiothérapie (12) et étant positionné à l'intérieur du faisceau de radiation (24), le détecteur de référence (20) générant un signal de référence ;
la normalisation du signal de mesure de faisceau sur la base du signal de référence, et
la production d'une mesure de faisceau normalisée ;
dans lequel
le détecteur de référence (20) est positionné à l'extérieur du faisceau de radiation (24) pendant le fonctionnement du dispositif de radiothérapie (12),
**caractérisé en ce que** le détecteur de référence (20) est porté sur le portique mobile afin de se déplacer avec celui-ci, dans lequel la tête (26) est configurée pour diriger le faisceau de radiation (24) éloigné d'un premier côté du portique mobile et le détecteur de référence (20) est porté sur un second côté du portique mobile opposé au premier côté.

2. Système de radiothérapie (10) selon la revendication 1, dans lequel le détecteur de référence (20) est positionné de manière à mesurer la dispersion de la tête (30).

3. Système de radiothérapie (10) selon les revendications 1 et 2, dans lequel la mesure de faisceau normalisée comprend une mesure d'une intensité relative du faisceau de radiation (24) en fonction de la position.

4. Système de radiothérapie (10) selon l'une quelconque des revendications 1 à 3, comprenant en outre : le détecteur de champ (16).

5. Système de radiothérapie (10) selon l'une quelconque des revendications 1 à 4, dans lequel le détecteur de champ (16) et le détecteur de référence (20) sont configurés pour détecter une radiation ionisante et dans lequel les signaux de mesure de faisceau et de référence sont représentatifs d'une charge acquise résultant de la détection de la radiation ionisante au niveau du détecteur de champ (16) et du détecteur de référence (20), respectivement.

6. Système de radiothérapie (10) selon l'une quelconque des revendications 1 à 5, dans lequel le matériel informatique comprend un processeur programmable et un support lisible par machine stockant des instructions qui, lorsqu'elles sont exécutées par le processeur programmable, amènent le processeur programmable à exécuter au moins certaines des opérations.

7. Procédé pour fournir une mesure de faisceau de radiation normalisée pour un dispositif de radiothérapie (12), le procédé comprenant :
la mesure d'un signal de mesure de faisceau représentatif d'une radiation ionisante à l'intérieur d'un faisceau de radiation (24) dirigé depuis une tête (26) du dispositif de radiothérapie (12) ;
la mesure d'un signal de référence représentatif d'une radiation ionisante ;
la normalisation du signal de mesure de faisceau sur la base du signal de référence pour générer la mesure de faisceau de radiation normalisée ; et
la production de la mesure de faisceau de radiation normalisée ;
**caractérisé en ce que**
le signal de référence est représentatif d'une radiation ionisante située à l'extérieur du faisceau de radiation (24), dans lequel le signal de référence est généré par un détecteur de référence (20) positionné à l'extérieur du faisceau de radiation (24) pendant le fonctionnement du dispositif de radiothérapie (12), dans lequel le dispositif de radiothérapie (12) comprend un portique mobile qui porte la tête (26) et le détecteur de référence (20) est porté sur le portique mobile afin de se déplacer avec celui-ci, dans lequel la tête (26) dirige le faisceau de radiation (24) éloigné d'un premier côté du portique mobile et le détecteur de référence (20) est porté sur un second côté du portique mobile opposé au premier côté.

8. Procédé selon la revendication 7, dans lequel le signal de mesure de faisceau est généré par un détecteur de champ (16) positionné à l'intérieur du faisceau de radiation (24) pendant le fonctionnement du dispositif de radiothérapie (12).

9. Procédé selon la revendication 8, dans lequel le détecteur de référence (20) est positionné de manière à recevoir une dispersion de la tête (30) provenant du dispositif de radiothérapie (12).

10. Procédé selon l'une quelconque des revendications 7 à 9, dans lequel le dispositif de radiothérapie (12) comprend un accélérateur linéaire (LINAC).

11. Procédé selon l'une quelconque des revendications 7 à 10, dans lequel la mesure de faisceau normalisée comprend une mesure d'une intensité relative du faisceau de radiation (24) en fonction de la position et/ou
la mesure des signaux de mesure de faisceau et de référence provenant du détecteur de champ (16) et du détecteur de référence (20) est effectuée à l'aide d'un électromètre qui produit les signaux de mesure de faisceau et de référence.

12. Produit de programme informatique comprenant un support lisible par machine stockant des instructions pour amener le système selon la revendication 1 à effectuer des opérations comprenant :
la réception d'un signal de mesure de faisceau provenant d'un détecteur de champ (16) et d'un signal de référence provenant d'un détecteur de référence (20), le détecteur de champ (16) générant un signal de mesure de faisceau représentatif d'un faisceau de radiation (24) dirigé depuis une tête d'un dispositif de radiothérapie pendant le fonctionnement du dispositif de radiothérapie (12) et étant positionné à l'intérieur du faisceau de radiation (24), le détecteur de référence (20) générant un signal de référence ;
la normalisation du signal de mesure de faisceau sur la base du signal de référence, et
la production d'une mesure de faisceau normalisée ;
**caractérisé en ce que**
le détecteur de référence (20) est positionné à l'extérieur du faisceau de radiation (24) pendant le fonctionnement du dispositif de radiothérapie (12), dans lequel le dispositif de radiothérapie (12) comprend un portique mobile qui porte la tête (26) et le détecteur de référence (20) est porté sur le portique mobile afin de se déplacer avec celui-ci, dans lequel la tête (26) dirige le faisceau de radiation (24) éloigné d'un premier côté du portique mobile et le détecteur de référence (20) est porté sur un second côté du portique mobile opposé au premier côté.
